**Europäisches Patentamt**

(19) **European Patent Office**

**Office européen des brevets**

(11) Veröffentlichungsnummer: **0 136 701**

**A2**

(12) **EUROPÄISCHE PATENTANMELDUNG**

(21) Anmeldenummer: **84111798.9**

(22) Anmeldetag: **03.10.84**

(51) Int. Cl.⁴: **G 01 N 11/00**

(30) Priorität: **05.10.83 DE 3336218**

(43) Veröffentlichungstag der Anmeldung:
**10.04.85 Patentblatt 85/15**

(84) Benannte Vertragsstaaten:
**AT BE CH DE FR GB IT LI NL SE**

(71) Anmelder: **Dierks & Söhne GmbH & Co. KG**
**Sandbachstrasse 1**
**D-4500 Osnabrück(DE)**

(72) Erfinder: **Ahlert, Dieter**
**Maschstrasse 2**
**D-4514 Ostercappeln 1(DE)**

(74) Vertreter: **Busse & Busse Patentanwälte**
**Postfach 1226 Grosshandelsring 6**
**D-4500 Osnabrück(DE)**

(54) **Messeinrichtung.**

(57) Eine Meßeinrichtung (1) für vor- bzw. innerhalb einer Wandung (9) bewegtes körniges oder körnige Partikel enthaltendes Gut, insbesondere in einem Behälter )3) schleudernd bewegtes Misch- oder Granuliergut, wobei ein an der Wandung (9) abgestützter, von der Wandung (9) in den Bewegungsraum des Guts vorstehender Fühler (12) über einen Fuß (17) auf zumindest einen elektrischen Meßaufnehmer (24 bis 29) einwirkt, wird im Sinne einer verbesserten Robustheit, vergrößerten Ansprechempfindlichkeit, einer ständigen Meßbereitschaft und einer geringeren Baugröße dadurch verbessert, daß der Fuß des Fühlers (12) als auslenkbare Platte (17) ausgebildet ist, die den Fühler (12) flächig mit der Wand verbindet und rückseitig den bzw. zumindest einen elektrischen Meßaufnehmer (24 bis 29) abdeckt.

Fig. 2

0136701

- 1 -                                  EB/Rh


Meßeinrichtung

Die Erfindung betrifft eine Meßeinrichtung nach dem Oberbegriff des Anspruchs 1,wie sie aus der DE-PS 26 53 864 vorbekannt ist. Derartige Meßeinrichtungen sind aufgrund ihrer Ansprechempfindlichkeit undihrer breitbandigen Signalaufnahme und -abgabe in der Lage, den Zustand des vorbeibewegten Guts, insbesondere des Misch-oder Granulierguts im Behälter hinsichtlich der Partikelgröße und -häufigkeit sowie hinsichtlich der Viskosität des Behälterinhalts zu unterscheiden, indem nicht nur ein Mittelwert für den vom vorbeibewegten Gut auf den Fühler ausgeübten "Staudruck", sondern ggf. auch ein Spektrum von Impulssignalen entsprechend dem Auftreffen von Partikeln ausgegeben wird.

Die vorbekannte Ausführungsform ist längsgestreckt aufgebaut, wobei ein Biegestab kopfseitig den Fühler trägt und fußseitig in einer seiner festen Einspannung benachbarten Biegezone Dehnungsmeßstreifen zur Umsetzung der mechanischen Biegespannungen in elektrische Signale trägt. Die Meßeinrichtung ist nach Art eines Teleskopstabs aufgebaut, wobei der Fühler mitsamt einem den Fuß des Fühlers und die an diesem angebrachten Meßelemente umschließenden Rohrteile in einer weiteren Hülse angeordnet sind, die ihrerseits mit der Wandung des Behälters verbunden ist. Dieser Aufbau der Meßeinrichtung berücksichtigt das in der Patentschrift unerwähnte praktische Erfordernis, den Fühler in die Hülse zurückziehen zu können, wenn keine Meßergebnisse angefordert sind. Damit kann die Gefahr einer Beschädigung
des exponierten Fühlers abgewendet werden, wenn etwa
brockiges Mischgut in den Behälter eingegeben wird. Entsprechend ist auch bei der in der Praxis vorbekannten Meßeinrichtung der Fühler am Deckel des Behälters befestigt, damit
er aus dem Bereich des Mischguts zurückziehbar ist.

Das Zurückziehen des Fühlers liefert aber lediglich einen
bedingten mechanischen Schutz, überdies bleibt der Fußbereich des Fühlers Verschmutzungen wie auch chemischen
und thermischen Einwirkungen ausgesetzt. Weiterhin ist das
teleskopartige Ausfahren des Fühlers in der Konstruktion
aufwendig und beim Messen umständlich, zumal mit Rücksicht
auf eine geschützte Rückzugslage eine Anordnung der Meßeinrichtung im Deckel des Behälters gewählt wird, dann aber
hinreichende Ausfahrlängen vorgesehen werden müssen, um in
das im Behälter bewegte Gut hineinreichen zu können.

Aufgabe der Erfindung ist es demgegenüber, eine Meßeinrichtung zu schaffen, welche die vorgenannten Schwierigkeiten einschneidend vermindert und bei hoher Betriebssicherheit einfache und schnelle Messungen erlaubt.

Gemäß der Erfindung wird dieses mit einer Meßeinrichtung nach
dem Anspruch 1 erreicht. Die Abkehr von dem generell stabförmigen Aufbau des Fühlers führt dazu, daß wenig mehr als
der eigentliche Fühlerkopf gegenüber der Wandung vorsteht,
während die für die Meßaufnahme vorzusehenden Verformungsbereiche einem plattenförmigen, vorzugsweise membranartigen
Fuß zugewiesen sind. Der plattenförmige Fuß läßt sich flach
an oder in der Wandung anordnen. Diese flache Anordnung ist
besonders dann wichtig, wenn das Gut im Behälter schleudernd
bewegt wird, etwa in Form einer von einem bodenseitigen Mischwerkzeug hervorgerufenen Trombenbewegung, bei der die Behälterwandung zur Bildung der Trombe beiträgt. Ein an die
Wandung angepaßter, plattenförmiger Fuß ermöglicht eine störungsfreie Ausbildung der Trombe.

Die reduzierten Abmessungen und Massen der Einrichtung verbessern die Ansprechempfindlichkeit des Fühlers und die Bandbreite des Signals, gleichzeitig führt die gedrängte Bauweise zur Unterdrückung störender Eigenschwingungen.

Mit der wandflachen Anordnung des plattenförmigen Fußes und mit der im wesentlichen auf den Fühlerkopf reduzierten Bauhöhe des in den Behälter hineinragenden Teils der Meßeinrichtung ergibt sich, daß diese wenig beansprucht wird und dementsprechend wie auch infolge des robusteren Aufbaus selbst gröberen Materialien im Behälter standzuhalten vermag. Der Fühler braucht keine Rückzugsstellung mehr. Damit kann die Meßeinrichtung ohne "bewegte Teile", d.h. ohne teleskopartig verfahrbare Teile, und auch ohne entsprechende Antriebe und Steuerungen ausgeführt werden. Die Meßeinrichtung wird einfacher und robuster und bleibt ständig einsatzbereit.

Die größere Robustheit der Meßeinrichtung erlaubt es auch, diese in einem Behälter von der früher üblichen Anordnung im Deckel in einen tieferliegenden Wandbereich zu verlegen, indem auch bei geringer Füllung des Behälters Mischgut oder Granuliergut anzutreffen ist. Bisher zu beachtende einschränkende Meßbedingungen entfallen, denen zufolge der Behälter einen vorgegebenen hohen Füllungsgrad aufweisen mußte, wenn der Fühler vom Gut ausreichend beaufschlagt werden sollte.

Die verformbare Platte bildet gleichzeitig eine Trennwand zwischen dem Fühlerkopf einerseits und dem Meßaufnehmer andererseits, wodurch letzterer mechanisch und chemisch geschützt ist. Grundsätzlich können verschiedene mechanisch-elektrische Signalwandler hinter der verformbaren Platte Verwendung finden. Vorzugsweise werden in dieser Hinsicht erprobte Dehnungsmeßstreifen-Elemente eingesetzt, die auf die Rückseite der verformbaren Platte geklebt werden und die Verformungen der Platte signalisiert.

Die verformbare Platte kann unterschiedliche Formen aufweisen, z.B. auch in einer Richtung gestreckte bandartige Formen, etwa bei von vornherein festliegender Beaufschlagungsrichtung für den Fühler. Vorzugsweise ist die verformbare Platte aber eine flächig ausgedehnte elastische Membran, wobei die flächige Ausdehnung die Anordnung verteilter Meßaufnehmer und dementsprechend auch die Auswertung der Signale hinsichtlich der Beaufschlagungsrichtung für den Fühler ermöglicht.

Weitere Merkmale und Vorteile der Erfindung ergeben sich aus den Ansprüchen und der nachfolgenden Beschreibung, in der ein Ausführungsbeispiel des Gegenstands der Erfindung anhand einer Zeichnung näher erläutert ist. In der Zeichnung zeigen:

Fig. 1     Seitenansicht eines Mischers mit Schnitt durch einen Mischbehälter,

Fig. 2     vergrößerte Detailansicht aus Fig. 1 unter Darstellung eines Teils des Mischbehälters mit Meßeinrichtung aus Fig. 1 und

Fig. 3     Schnitt nach Linie III-III in Fig. 2.

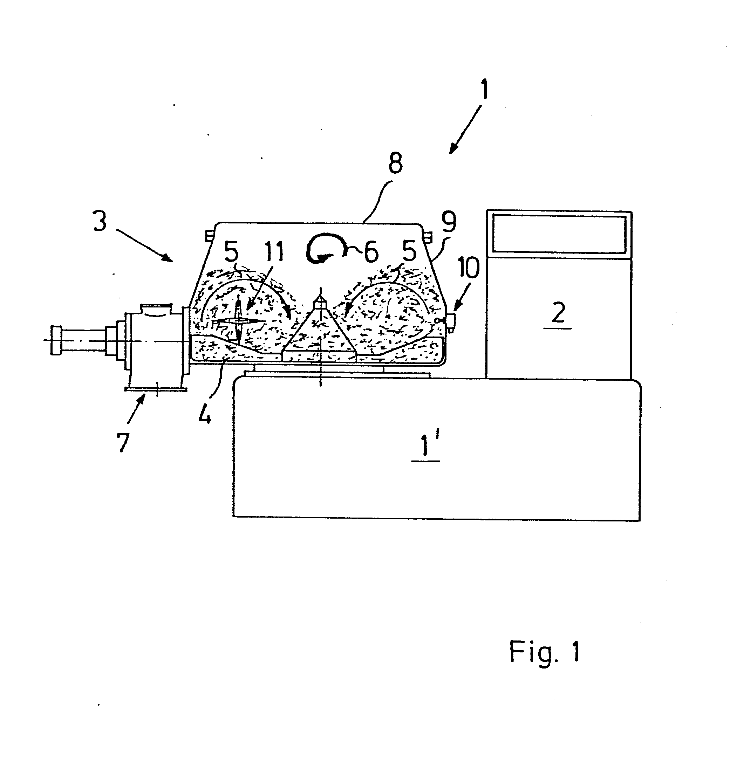

In Fig. 1 ist ein Mischer weitgehend bekannter Art dargestellt und insgesamt mit 1 bezeichnet. Der Mischer steht mit einem Gehäuse 1' auf dem Boden auf, welches ein Schaltpult 2 und einen Behälter 3 trägt, in dem ein Mischwerkzeug 4 mit relativ hoher Geschwindigkeit derart rotieren kann, daß das im Behälter 3 enthaltene Gut eine trombenförmige Bewegung ausführt. Diese Bewegung umfaßt das außenseitige Aufsteigen des Guts und Zurückfallen in einem inneren Bereich entsprechend den Pfeilen 5 wie auch das Umlaufen mit dem Mischwerkzeug 4 um die Behälterachse entsprechend einem Pfeil 6. Bei 7 ist eine Entleerungsvorrichtung angedeutet, wie sie bei solchen Behältern neben verschiedensten Einrichtungen zur Zufuhr oder Abfuhr von Materialien, Flüssigkeiten, Gasen oder zur Beaufschlagung mit Wärme, Vakuum, Druck usw. üblich sind.

Der unterseitig zunächst zylindrisch ansteigende und sich dann konisch zu einem Behälterdeckel 8 verengende Mantel 9 des Behälters 3 enthält eine insgesamt mit 10 bezeichnete Meßeinrichtung, die im folgenden näher beschrieben wird. Die Anordnung dieser Meßeinrichtung liegt noch im unteren, zylindrischen Teil des Behältermantels 9, dicht oberhalb der Bewegungsbahn des Mischwerkzeugs 4.

In Umlaufrichtung des Mischwerkzeugs 4 betrachtet ist die Meßeinrichtung 10 unter einem Winkel von etwa 140° vor einem Zerkleinerungswerkzeug 11 bekannter Art angeordnet, welches gleichfalls im unteren Bereich des Behältermantels 9 gelagert ist und von außen in den Behälter hineinragt. Die Anordnung der Meßeinrichtung vor dem Zerkleinerungswerkzeug, und zwar mit Abstand vor diesem, hat sich als vorteilhaft herausgestellt, da das Zerkleinerungswerkzeug die trombenförmige Bewegung des Mischguts beeinflußt, wobei dieser Einfluß längs des Umfangs (in Umlaufrichtung des Mischwerkzeugs und des Gutes) stetig abnimmt, um kurz vor dem Zerkleinerungswerkzeug wieder infolge örtlicher Rückwirkung anzusteigen. Um den im allgemeinen als Störgröße zu betrachtenden Einfluß des Zerkleinerungswerkzeugs 11 möglichst zu eliminieren, ist das Mischwerkzeug 10 im Winkelabstand von etwa 140° vor dem Werkzeug angeordnet.

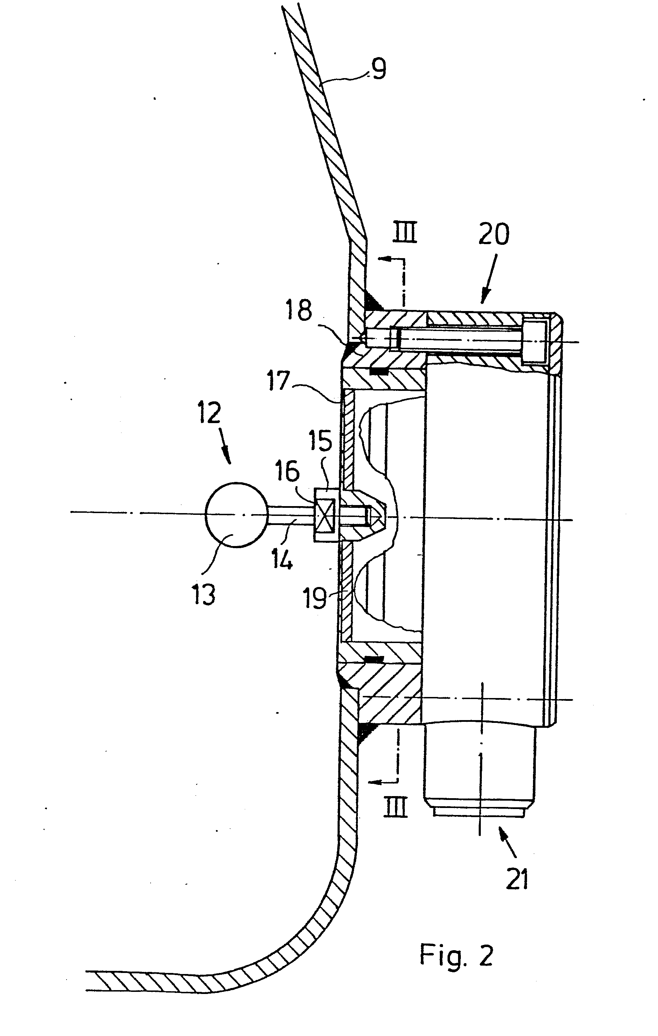

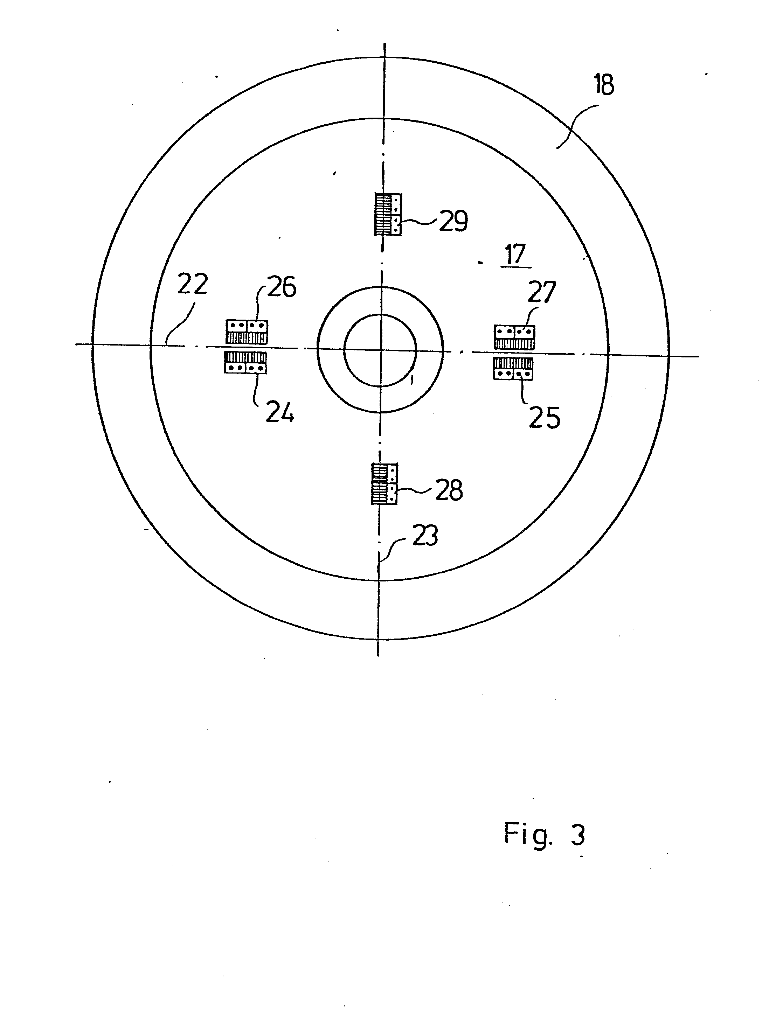

In Fig. 2 ist in der vergrößerten Darstellung ein Bereich der Behälterwand 9 zu erkennen, gegenüber der zum Behälterinneren hin ein kurzer Fühler 12 mit kugelförmigem Kopf 13 und zylindrischem Hals 14 vorsteht. Diese Konfiguration ist massearm, um auf das Auftreffen feiner Partikel anzusprechen, sie

0136701

ist aber auch hinreichend robust, um Aufschlägen massiveren Guts zu widerstehen. Der Kugelkopf bietet dem Gut im Behälter eine strömungsgünstige und damit wenig verschmutzungsanfällige Form, wobei auch der dünnere Hals 14 das Festsetzen von Verschmutzungen zur Wandung hin unterdrückt. Der Fühler 12 ist über einen Schraubansatz 15 mit Schlüsselflächen 16 in einer verformbaren Metallplatte oder Membran 17 mittig befestigt, wobei die Platte 17 - wie aus Fig. 3 noch ersichtlich sein wird - eine kreisförmige Gestalt besitzt. Die Platte 17 ist randseitig plan zur Behälterwand gehalten und trägt in der Mitte eine Schraubaufnahme 18 für den Fühler 12. Der Schraubansatz ermöglicht das Auswechseln des Fühlers und den wahlweisen Einsatz von Fühlern unterschiedlicher Größe und/oder Masse, er kann aber auch zugunsten einer festen Verbindung entfallen.

Wird in dem Behälter 3 eine Mischbewegung erzeugt, indem pastöses oder körniges Gut auf den Fühler 12 auftrifft, dann wird dieser je nach Art der Beanspruchung gleichförmig oder auch impulsförmig ausgelenkt, wobei die Auslenkung von der Platte 17 aufgefangen wird, die sich dabei verformt.

Auf der Rückseite der Platte 17 und von dieser geschützt sind innerhalb einer Vergußschicht 19 Dehnungsmeßstreifen angeordnet, die die Verformungen der Platte 17 messen und an eine nicht näher dargestellte aber grundsätzlich bekannte Auswerteeinrichtung weitergeben. Diese Auswerteeinrichtung liegt in einem vom Behälter 3 getrennten Gehäuse. Am Behälter befindet sich nur die eigentliche Meßeinrichtung mit einem Gehäuse 20, welches in eine Kabeldurchführung 21 ausmündet.

Die Fig. 3 zeigt eine rückseitige Ansicht der verformbaren Platte 17 mit der genaueren Anordnung von Dehnungsmeßstreifen (allerdings ohne deren Verdrahtung). Die kreisförmige Platte 17 ist mit zwei senkrecht zueinanderstehenden Durchmesserlinien 22 und 23 aufgeteilt, längs derer Dehnungsmeßstreifen angeordnet sind. Eine Hauptrichtung wird dabei durch die Durchmesserlinie 22 bestimmt, an dieser sind vier Dehnungsmeßstreifen 24,25 und 26,27 angeordnet. Diese vier Dehnungsmeßstreifen-Elemente liegen einander paarweise auf gleichem Durchmesser gegenüber, wobei die Dehnungsmeßstreifen 24 und 26 sowie die Dehnungsmeßstreifen 25 und 27 dicht aneinander gerückt sind, um innerhalb einer Brückenschaltung zur Temperaturkompensation paarweise ausgewertet zu werden.

Wird der Fühler 12 längs der Durchmesserlinie 22 stoßförmig oder gleichförmig beansprucht, dann erfahren die Dehnungsmeßstreifenpaare unterschiedliche Verformungen und geben dementsprechend gegenläufige Signalveränderungen als Maß der Beanspruchung wieder, wobei die Frequenzcharakteristik dieser Dehnungsmeßstreifen auch impulsförmige Signale weitergibt.

Längs der Durchmesserlinie 23 sind zwei Dehnungsmeßstreifen 28 und 29 einander diametral gegenüberliegend angeordnet. Diese Dehnungsmeßstreifen sprechen nicht an, wenn der Fühler 12 eine Beanspruchung in Richtung der Durchmesserlinie 22 erfährt, sondern nur dann, wenn zumindest eine Richtungskomponente der Beanspruchung auf die Durchmesserlinie 23 fällt. Mit einer solchen Ausgestaltung ist es möglich, Richtungsänderungen zu unterdrücken, indem die Beanspruchungen komponentengerecht zusammengesetzt werden. Es ist aber auch möglich, die Richtung der Beanspruchung zu ermitteln, was einen zusätzlichen Aufschluß über die Bewegung des Mischguts im Behälter liefert. Auch dieses ist für die Auswertung geeignet und nützlich.

Die beschriebene Meßvorrichtung schafft bei aller Robustheit eine feinfühlige, stetige Überwachung des Mischguts im Behälter, solange dieses bewegt wird. Damit läßt sich die bisher schon erfolgreich eingesetzte Überwachung des Mischguts insbesondere bei Arbeitsverfahren im pharmazeutischen Bereich mit hoher Genauigkeit und Reproduzierbarkeit verbessern, vereinfachen und zu einer ständigen Überwachung ausdehnen. Die wandflache Anordnung mit geringer Bauhöhe und geringer Fühlermasse erlaubt den Verzicht auf bewegte Teile mitsamt Antrieben und Steuerungen, die Anordnung im unteren Behälterbereich mit Meßmöglichkeit auch bei nur teilweise gefülltem Behälter und den Verzicht auf besondere Wartungs-und Prüfungsmaßnahmen hinsichtlich der Behälterabdichtung im Bereich der Meßeinrichtung, nachdem keine Bewegungsspalte mehr vorzusehen sind.

Auch die Montage und das bedarfsweise Auswechseln der Meßeinrichtung ist, wie aus Fig. 2 noch ersichtlich, einfach gestaltet. Die Meßeinrichtung weist einen gegenüber dem Gehäuse 20 vorspringenden Stutzen 30 auf, der von außen in einen behälterfesten Befestigungsring 31 eingeschoben ist und diesem gegenüber noch zusätzlich über eine Ringdichtung 32 abgedichtet wird. Befestigungsschrauben 33 zwischem dem Gehäuse 20 und dem Befestigungsring 31 sichern die Meßeinrichtung in ihrer Einbaulage, aus der sie jederzeit wieder entnommen werden kann, sei es zum Austausch und sei es auch nur zu einer gründlichen Reinigung.

- 9 -

EB/Rh

Patentansprüche:

1. Meßeinrichtung für vor- bzw. innerhalb einer Wandung bewegtes körniges oder körnige Partikel enthaltendes Gut, insbesondere in einem Behälter schleudernd bewegtes Misch- oder Granuliergut, wobei ein an der Wandung abgestützter, von der Wandung in den Bewegungsraum des Guts vorstehender Fühler über einen Fuß auf zumindest einen elektrischen Meßaufnehmer einwirkt, dadurch gekennzeichnet, daß der Fuß des Fühlers als auslenkbare Platte (17) ausgebildet ist, die den Fühler (12) flächig mit der Wand verbindet und rückseitig den bzw. zumindest einen elektrischen Meßaufnehmer (24 bis 29) abdeckt.

2. Meßeinrichtung nach Anspruch 1, dadurch gekennzeichnet, daß die auslenkbare Platte (17) eine elastische Membran ist.

3. Meßeinrichtung nach Anspruch 2, dadurch gekennzeichnet, daß die Meßaufnehmer (24 bis 29) unmittelbar auf der Rückseite der Membran (17) angeordnete Dehnungsmeßstreifen sind.

4. Meßeinrichtung nach Anspruch 3, dadurch gekennzeichnet, daß zumindest zwei Meßaufnehmer (24,25;26,27;28,29) einander bezüglich des Fühlers (12) diametral und abstandsgleich gegenüberliegend angeordnet sind.

5. Meßeinrichtung nach Anspruch 3 oder 4, dadurch gekennzeichnet, daß die Membran (17) kreisförmig ausgebildet ist und auf zumindest zwei verschiedenen Durchmesserlinien (22,23) angeordnete Meßaufnehmer (24 bis 29) trägt.

6.      Meßeinrichtung nach einem der Ansprüche 1 bis 5, an einem Mischbehälter mit einem bodenseitig schnell umlaufenden, eine trombenförmige Mischbewegung erzeugenden Mischwerkzeug, dadurch gekennzeichnet, daß der Fühler (12) feststehend an der Seitenwandung (9) des Behälters (3) angeordnet ist.

7.      Meßeinrichtung nach Anspruch 6, wobei der Mischbehälter ein von der Seitenwandung in den Behälter hineingreifendes Zerhackerwerkzeug besitzt, dadurch gekennzeichnet, daß die Meßeinrichtung (10) in Umlaufrichtung vor dem Zerhackerwerkzeug (11) angeordnet ist.

8.      Meßeinrichtung nach Anspruch 7, dadurch gekennzeichnet, daß die Meßeinrichtung (10) bezüglich der Umlaufrichtung etwa 140° vor dem Zerkackerwerkzeug (11) angeordnet ist.

Fig. 1

0136701

Fig. 2

0136701

Fig. 3